# EUROPEAN PATENT APPLICATION

(11) **EP 4 411 375 A1**
(43) Date of publication of application: **07.08.2024**
(21) Application number: 23154713.4
(22) Date of filing: 02.02.2023
(51) Int. Cl.: G01N 33/50, C40B 30/06

(54) **IMPROVED METHOD AND SYSTEM FOR DETERMINING EFFICACIES FOR ONE OR MORE PHARMACEUTICAL DRUGS FOR A PLURALITY OF MEDICAL INDICATIONS**

(71) Applicant: Precomb Therapeutics AG, 8634 Hombrechtikon (CH)
(72) Inventor: Mauti, Olivier, CH-8005 Zürich (CH); Steiner, Peter, CH-8852 Altendorf (CH); Kelm, Jens, CH-8610 Uster (CH)
(74) Representative: Krauss, Jan

(57) **Abstract**

The present invention relates to an improved method for determining efficacies of one or more drugs for a plurality of indications, based on data obtained from a plurality of patients using standardized and integrated testing systems, as well as respective systems for performing the method, and the analysis and storage of the data as generated.

## Description

The present invention relates to an improved method for determining efficacies of one or more drugs for a plurality of indications, based on data obtained from a plurality of patients using standardized and integrated testing systems, as well as respective systems for performing the method, and the analysis and storage of the data as generated.

### Background of the invention

While pharmaceutical preparations are usually registered and available as standard dosage forms, in reality the efficacy of pharmaceutically active compounds for an individual patient is highly diverse, and depends on a multitude of factors, like age, weight, and metabolism of a patient, to name only a few.

Furthermore, attempts to analyze efficiencies in order to identify the most promising and least burdensome therapy for an individual patient having a specific disease or condition are aggravated by a lack of standardized high-quality data collection methods and systems, and therefore today many more complex therapies, like the chemotherapeutic treatment of a specific cancer, are still largely trial and error.

Medical researchers can use large amounts of data on treatment plans and recovery rates of cancer patients in order to find trends and treatments that have the highest rates of success in the real world. For example, researchers can examine tumor samples in biobanks that are linked up with patient treatment records. Using this "big" data, researchers can see things like how certain mutations and cancer proteins interact with different treatments and find trends that will lead to better outcomes.

An interesting example of the use of big data in healthcare is the Cancer Moonshot program. Before the end of his second term, President Obama came up with this program that had the goal of accomplishing 10 years' worth of progress towards curing cancer in half that time. However, in order to make these kinds of insights more available, patient databases from different institutions such as hospitals, universities, and nonprofits need to be linked up. Then, for example, researchers could access patient biopsy reports from other institutions. One of the potential big data use cases in healthcare could be genetically sequencing cancer tissue samples from clinical trial patients and making these data available to the wider cancer database.

The goal of healthcare online business intelligence is to help doctors make data-driven decisions within seconds and improve patients' treatment. This is particularly useful in the case of patients with complex medical histories, suffering from multiple conditions. New solutions and tools would also be able to predict, for example, who is at risk of diabetes and thereby be advised to make use of additional screenings or weight management.

Some studies have shown that 93% of healthcare organizations have experienced a data breach. The reason is simple: personal data is extremely valuable and profitable on the black markets. And any breach would have dramatic consequences.

Thus, currently there are quite a few of obstacles in the way, including:
- Incompatible data systems. This is perhaps the biggest technical challenge, as making these data sets able to interface with each other is quite a feat.
- Patient confidentiality issues. There are also differing laws which govern what patient information can be released with or without consent, and all of these would have to be navigated.
- Institutions that have put a lot of resources and money into developing their own cancer dataset may not be eager to share it with others, even though it could lead to a cure much more quickly.

While devices and methods to determine the efficacy of pharmaceutically active compounds for an individual patient are known, e.g., from WO 2021/110799 or DE 10 2022 100 146.6 and methods are known, e.g, from US 8,265,954 B2, to extract and normalize health care data from patients using a computer system, methods and systems are still missing that provide a solution for the above needs regarding an efficient and effective treatment of individual patients.

It is therefore an object of the present invention to provide such new and improved methods and systems. Other objects and advantages of the present invention will become apparent to the person of skill when studying the following more detailed description of the present invention, including the Figures and examples.

In a first aspect of the present invention, the above object is solved by an improved method for determining efficacies for one or more pharmaceutical drugs for a plurality of medical indications, the method comprising
i) obtaining raw data regarding the effect of one or more pharmaceutical drugs for a plurality of patients that are afflicted by at least one of the plurality of medical indications, at a plurality of separate testing sites or centers, and using standardized testing systems,
   wherein said obtaining comprises the steps of:
   - providing cells from at least one patient-derived tissue sample;
   - using the standardized testing system, generating 3D microtissues in said system based on the cells as provided;
   - contacting the 3D microtissues as generated with the one or more pharmaceutical drugs to be tested;
   - collecting the raw data regarding the effect of the one or more pharmaceutical drugs on the 3D microtissues; and
ii) transmitting the raw data as collected to a centralized server; and
iii) based on the raw effect data as received at the centralized server, determining the efficacies for the one or more pharmaceutical drugs as tested for at least one medical indication, and preferably for a plurality of medical indications, at the centralized server.

Preferred is a method according to the present invention, further comprising the step of selecting the pharmaceutical drug as determined having the highest efficacy for at least one medical indication, wherein more preferably the efficacy is patient-specific. Further preferred is the method according to the present invention, wherein a combination of pharmaceutical drugs is selected as determined having the highest efficacy for at least one medical indication.

Preferred is a method according to the present invention, comprising a plurality (2, 3, 4, or more) of standardized testing systems transmitting raw data as collected to the centralized server, wherein the standardized systems are positioned at the same or different locations.

In order to address the shortcomings of the currently used medical data and data gathering infrastructure with respect to determining efficacies for one or more pharmaceutical drugs for a plurality of medical indications in patients, in particular in the field of chemotherapeutic and targeted interventions, the present inventors designed the improved methods and systems according to the present invention.

At the core of the invention is the strategy to provide a method that requires as little "human factor intervention" as possible. Ideally, the patient tissue sample as obtained is introduced into an autonomous data generation system ("standardized testing system") located at the test center, and no more human intervention required with respect to the sample. This reduces/excludes any personal bias stemming from the handling of samples by the involved personnel.

The standardized testing system will be able to autonomously generate standardized and thus highly compatible raw data regarding the effect of the one or more pharmaceutical drugs on the 3D microtissues as automatically generated.

At the local test center, only media, media comprising the drugs to be tested, and other consumed materials (well-plates, etc.) will have to be supplied as required. Preferably, these are supplied as ready-to-use supply packs or so-called preconfigured containers that can be fed into the standardized testing system with minimal handling. In a preferred embodiment, the standardized testing system thus includes suitable means to introduce these packs, like sterile ports, robotic handling arms, a loading system with a lock system, and the like.

The personnel located at the test center will then optionally have to put in additional and/or "secondary" data into an interface of standardized testing system. Again, advantageously this is done with minimal manual handling, for example using a bar code reader reading bar code-encoded test samples. Alternatively or additionally, the standardized testing system will include means to automatically administer sample material as introduced into the system, in order to exclude mix-ups and other errors.

Preferred is the method according to the present invention, wherein the raw data as collected at the plurality of separate testing centers is transmitted to the centralized server together with patient-specific secondary data, wherein preferably the patient-specific secondary data comprise one or more of: an electronic health record of the patient, genomic data of the patient, and/or data relating to one or more biomarkers of the patient as analyzed or co-analyzed.

The standardized testing system comprises means to generate 3D microtissues in said system based on the cells or tissues as provided. The system as described in, for example, WO 2021/110799, can be readily adjusted to the requirements of the method as described herein, e.g. by providing it with a digital interface to run the individual protocols and transfer the raw or production data to the external data system as herein for further data assessment.

In the method according to the present invention, the at least one patient-derived tissue sample can be selected from a sub-sample derived from a primary tissue sample, a primary tumor sample, and a metastasis sample, wherein preferably said tissue sample has been obtained by a method comprising core biopsy, tumor resection, liquid biopsy and/or needle aspiration, and/or wherein said tissue sample and/or the dissociated cells are frozen and re-thawed prior to the generation of said 3D microtissues.

The method according to the present invention involves the suitable dissociation of the tissues and/or cells and the subsequent generation of 3D microtissues in said system, and the layout of the system is provided accordingly. Dissociating said tissue sample may comprise i) if required, dissecting said tissue sample into smaller pieces comprising cells, ii) treating said tissue sample with a solution comprising at least one enzyme capable of dissociating cells in said tissue sample, preferably at least one enzyme selected from a protease, a collagenase, trypsin, elastase, hyaluronidase, papain, chymotrypsin, deoxyribonuclease I, and neutral protease (dispase), producing a supernatant comprising dissociated cells, and ii) removing said supernatant comprises said dissociated cells and suitably collecting said cells, wherein steps (ii) and (iii) are repeated at least once, wherein preferably before step (ii) said tissue sample is sonicated with ultrasound, wherein the energy of said ultrasound is set at a suitable level to not destroy a substantial amount of said cells.

Generating 3D microtissues or microtumors in said system based on the cells as provided may comprise adding or removing stroma cells, stromal fibroblasts, endothelial cells and immune cells to said dissociated cells. For each 3D microtissue, a predetermined number of cells may be provided, such as, for example, between 500 and 10000 viable cells. Furthermore, the 3D microtissues may be generated in at least one system selected from a hanging drop system or a multiwell system, preferably comprising Ultra Low Adherence (ULA) wells, and the system is equipped accordingly. The generation of the 3D microtissues may comprises a maturation time of about 6 hours to 7 days, preferably about 1 to 6 days, more preferably about 2 to 5 days, and/or wherein said 3D microtissues as generated have a size of 350 um +/- 100 um.

After the 3D microtissues, such as microtumors, have been generated based on the cells or tissues as provided in the system, the 3D microtissues are contacted with the one or more pharmaceutical drugs to be tested. Preferred is the method according to the present invention, wherein the pharmaceutical drugs to be tested are supplied as ready-to-use supply packs or so-called preconfigured containers that can be fed into the standardized testing system with minimal handling. In a preferred embodiment, the standardized testing system thus includes suitable means to introduce these packs, like sterile ports, robotic handling arms, a loading system with a lock system, and the like. More preferably, the preconfigured container comprises a multi-well plate, further preferably having pre-defined concentrations of the one or more drugs at predefined locations. Preferred is the method according to the present invention, wherein the contacting the 3D microtissues with the one or more pharmaceutical drugs to be tested is performed by placing the 3D microtissues at these predefined locations.

In one aspect of the method according to the present invention, said contacting may comprises a continuous exposure to the pharmaceutical drugs to be tested and/or combinations thereof, and/or a cycle of an exposure to and subsequent removal to said pharmaceutical drugs to be tested and/or combinations thereof, optionally for at least one, preferably two and more cycles.

In another aspect of the method according to the present invention, the pharmaceutical drugs to be tested or combinations thereof that the 3D microtissues are contacted with are selected from the group consisting of cytotoxic, cytostatic and/or chemotherapeutic agents, targeted drugs, immunotherapeutic agents and/or combinations thereof. Examples for cytotoxic, cytostatic and/or chemotherapeutic agents are anastrozole, azathioprine, bcg, bicalutamide, chloramphenicol, ciclosporin, cidofovir, coal tar containing products, colchicine, danazol, diethylstilbestrol, dinoprostone, dithranol containing products, dutasteride, estradiol, exemestane, finasteride, flutamide, ganciclovir, gonadotrophin, chorionic, goserelin, interferon containing products (including peg-interferon), leflunomide, letrozole, leuprorelin acetate, medroxyprogesterone, megestrol, menotropins, mifepristone, mycophenolate mofetil, nafarelin, estrogen containing products, oxytocin (including syntocinon and syntometrine), podophyllyn, progesterone containing products, raloxifene, ribavirin, sirolimus, streptozocin, tacrolimus, tamoxifen, testosterone, thalidomide, toremifene, trifluridine, triptorelin, valganciclovir, and zidovudine. Targeted drugs are medications that increase in concentration in some parts of the body relative to others, such as antibodies. Examples are bevacizumab, everolimus in brain cancer; bevacizumab, everolimus, lapatinib, pertuzumab, trastuzumab and its antibody drug conjugates in breast cancer; aflibercept, bevacizumab, cetuximab, panitumumab, regorafenib in colorectal cancer; and imatinib in dermatofibrosarcoma protuberans. Immunotherapeutic agents are used in immunotherapy as a form of cancer treatment that uses the power of the body's immune system to prevent, control, and eliminate cancer. Examples are monoclonal antibodies to treat cancer, CAR T-cell therapy, immune checkpoint inhibitors to treat cancer, cancer peptide vaccines, immunomodulating drugs (IMiDs), and cytokines. In the context of the present invention, the term "pharmaceutical drugs", in addition to the drugs are above, relates to compounds that are used or can be used for the treatment of a disease or condition in a mammalian subject, such as a human patient.

Preferably, the pharmaceutical drugs to be tested are selected from anti-cancer drugs, such as, for example, alkylating agents, antimetabolites, natural products, hormones, tyrosine inhibitors, chemotherapeutic compounds, anti-cancer antibodies, in particular gemcitabine, abraxame, trametinib, olaparib, oxaliplatin, erlotinib, erlotinib, 5-FU, docetaxel, and pemetrexed.

As mentioned above, preferred is the method according to the present invention, wherein the pharmaceutical drugs to be tested are provided to the test as a pre-manufactured drug matrix, for example as a preconfigured container comprising a multi-well plate having pre-defined concentrations of the one or more drugs at predefined locations thereof.

Further preferred is the method according to the present invention, wherein the preconfigured container furthermore comprises a temperature logger configured to log a temperature of the preconfigured container over a, optionally predetermined, period of time, wherein preferably the temperature logger is mounted at a container that comprises the preconfigured multi-well plate.

In another embodiment of the method according to the present invention, the one or more drugs to be tested comprise one or more standardized concentrations of reference drugs and one or more candidate drugs to be tested.

In another embodiment of the method according to the present invention, the preconfigured multi-well plate comprises a cytotoxic agent at one or more predetermined control locations, and preferably the method comprises the additional step of analyzing whether the cells at the control locations of the preconfigured multi-well plate comprise dead cells at the one or more predetermined control locations as a positive control. Otherwise, the data obtained based on the preconfigured multi-well plate is regarded as invalid.

In the next step of the method according to the present invention, the raw data regarding the effect of the one or more pharmaceutical drugs to be tested on the 3D microtissues is collected. Collecting the data regarding the effect may comprise determining a physiological parameter selected from a size determination of said 3D microtissue, quantification of internal reporter gene expression in said 3D microtissue, determination of the intracellular ATP content in said 3D microtissue, and determination of pre-selected biomarkers in said 3D microtissue, wherein preferably said size determination of said 3D microtissue comprises at least one parameter selected from diameter, perimeter, volume, and area of optical cross section, and wherein preferably said size determination of said 3D microtissue comprises the use of an imaging device, and optionally further comprising the analysis of growth-kinetics.

The method according to the present invention can further comprise collecting and/or storing of the raw and/or secondary data in at least one local database present in the standardized system present at the local center. The data may be selected from the group consisting of data with respect to the pre-selection and/or grouping of the combination or panel of drugs, physiological parameters, data with respect to the effect(s) of said drugs or combination or panel or group of drugs thereof, data with respect to adverse effects, data with respect to additional clinical parameters, such as patient specific data and/or treatment history, data with respect to the stratification and/or monitoring, and data for automatization of the standardized system, for example for controlling at least one robot thereof. The data may be encrypted.

Preferred is a method according to the present invention, wherein the plurality of medical indications includes at least one of cancer, vascular diseases, lung diseases, autoimmune diseases, brain diseases, metabolic diseases, liver diseases, infectious diseases, such as bacterial or viral infections.

Preferred is a method according to the present invention, wherein the data is patient-specific, i.e., derived from and/or assigned to one patient, and/or one patient sample, such as the 3D microtissue or -tumor.

One or more, and preferably all of the above parameters are then used as raw data and are transmitted from the local data base of the standardized system to a centralized server. The data may be encrypted for the transfer. The transfer may be directly between the standardized system(s) and the centralized server or via an indirect transfer, e.g., the internet or other network structure. In a preferred embodiment of the method according to the present invention, the data is pseudomized and directly transmitted into the cloud without additional manual interaction by the local user. Preferred is the data transfer using a virtual private network (VPN).

In the next step of the method according to the present invention, based on the data as received at the centralized server the efficacy for the one or more pharmaceutical drugs as tested for at least one medical indication or for a multitude of medical indication is determined at the centralized server. Similar to the above, preferred is a method according to the present invention, wherein the plurality of medical indications includes at least one of cancer, vascular diseases, lung diseases, brain diseases, metabolic diseases, autoimmune diseases, liver diseases, infectious diseases, such as bacterial or viral infections. Also preferred is a method according to the present invention, wherein the data as determined is patient-specific, i.e., derived from and/or assigned to one patient, and/or one patient sample, such as the 3D microtissue or -tumor.

Further preferred is the determination, wherein the plurality of medical indications includes one, two, three or four different medical indications, e.g., different types of cancer or other proliferative diseases.

Further preferred is the determination, wherein the data as received and used for the determination is specific for a group of patients, e.g., different patients suffering from the same type of disease or medical indication, such as a cancer or other proliferative diseases, or a group of patients that have been assigned to the same or substantially the same therapeutic intervention, such as a chemotherapy.

According to the method according to the present invention, the efficacy for the one or more pharmaceutical drugs as tested for at least one medical indication or for a multitude of medical indication is determined comprising the steps of the introduction of the raw (and secondary) data into the server, a step of curation (organization and integration of data collected from various sources), (further) standardization, and quality control (QC) of the data, before the actual data analysis and interpretation, preferably comprising the use of suitable artificial intelligence (AI) software, and/or machine learning (ML) software (see, for example, Paul D, Sanap G, Shenoy S, Kalyane D, Kalia K, Tekade RK. Artificial intelligence in drug discovery and development. Drug Discov Today. 2021 Jan;26(1):80-93. doi: 10.1016/j.drudis.2020.10.010. Epub 2020 Oct 21. PMID: 33099022; PMCID: PMC7577280; Wang Y, Jeon H. 3D cell cultures toward quantitative high-throughput drug screening. Trends Pharmacol Sci. 2022 Jul;43(7):569-581. doi: 10.1016/j.tips.2022.03.014. Epub 2022 Apr 30. PMID: 35504760).

The centralized server may be linked to other information sources providing additional medical and/or scientific and/or pharmaceutical data ("third party information"), in order to improve and/or complete the determination as required.

Before an output is generated from the centralized server, the data processing may comprise additional steps of standardization, quality control (QC) of the output data, and may include automated failure alerts, for example if the quality of the raw data as provided is insufficient in order to determine a statistical meaningful result, as well as respective feedback(s) to operators. The data processing according to the method according to the present invention may also comprise the use of a data clustering algorithm (see, for example, Pina A, Macedo MP, Henriques R. Clustering Clinical Data in R. Methods Mol Biol. 2020;2051:309-343. doi: 10.1007/978-1-4939-9744-2_14. PMID: 31552636).

Preferably, the output as provided from the centralized server comprises an interactive cloud-based reporting to customers, e.g., as present in the local centers. The output may be direct to the customer, or indirect through the centralized system(s) as used to generate the raw data. Preferred is the data transfer using a virtual private network (VPN). Preferably, the output data is encrypted.

Preferred is a method according to the present invention, further comprising a step of obtaining, at the server, information, such as a feedback, on the therapeutic success of the one or more pharmaceutical drugs.

In the context of the present invention, the term "efficacy" or "efficacies" shall mean the ability of a specific therapeutic or preventive medical intervention, here the administration of at least one pharmaceutical drug for the prevention or treatment of at least one or a plurality of medical indications in a patient or a group of patients, to positively influence or to prevent a medical indication (disease or condition), also called "therapeutic success". In the context of the present invention, this ability is determined in the standardized system(s) under specific and controlled conditions, i.e., patient-specific and/or specific for a defined group of patients, e.g., different patients suffering from the same type of disease or medical indication, such as a cancer or other proliferative diseases, or a group of patients that have been assigned to the same or substantially the same therapeutic intervention, such as a chemotherapy.

As mentioned above, at the core of the invention is still the strategy to provide a method that requires as little "human factor intervention" as possible. The same strategy is applied to the centralized server, which autonomously determines and thus generates data with respect to the efficacies for the one or more pharmaceutical drugs as tested for a plurality of medical indications, and again no substantial human intervention is required with respect to the determination. Again, this reduces/excludes any personal bias stemming from the handling of data and interpretation thereof.

Another aspect of the present invention then relates to a method to generate a database and/or data platform comprising data with respect to the efficacies for the one or more pharmaceutical drugs as tested for a plurality of medical indications, comprising performing the method according to the present invention as above on a centralized server that autonomously determines and generates the data with respect to the efficacies for the one or more pharmaceutical drugs as tested for a plurality of medical indications, and the step of storing said data as generated in a database and/or data platform.

The centralized server may be linked to other information sources providing additional secondary data as disclosed herein and/or medical and/or scientific and/or pharmaceutical data ("third party information"), in order to improve and/or complete the data as stored in said database and/or data platform. The data may be selected from the group consisting of data with respect to the pre-selection and/or grouping of the combination or panel of drugs, physiological parameters, data with respect to the effect(s) of said drugs or combination or panel or group of drugs thereof, data with respect to adverse effects, data with respect to additional clinical parameters, such as patient specific data and/or treatment history, and data with respect to the stratification and/or medical monitoring.

Preferred is a method according to the present invention, wherein the data is patient-specific, i.e., derived from and/or assigned to one patient, and/or one patient sample, such as the 3D microtissue or -tumor.

The database and/or data platform may be present on the centralized server itself or a separate server or in the cloud. Preferably, the data in the database is encrypted.

Compared with the state of the art, the methods according to the present invention have several advantages. Overall, a reliable process is provided that avoids human bias, and thus provides highly significant and comparable data. This ultimately renders both processes of the identification of efficient chemotherapeutic drugs for certain indications as well as the development of more effective and efficient therapies faster, and also cheaper. Since in many proliferative diseases time is of the essence, this will also result in better patient outcome.

Furthermore, while the method according to the present invention involves a standardized and thus "integrated" system, which is largely closed to external interaction that could interfere with the steps of obtaining the raw data, in another preferred embodiment of the method, secondary and/or "foreign" relevant data can be integrated into the analysis as well. Nevertheless, since the core function of the system is largely closed to external interaction, "distortion" or "contamination" of the core functional data is avoided, which maintains the high significance of the data and results as provided.

Another aspect of the present invention then relates to a method to produce a pharmaceutical composition having improved efficacy for, preferably the personalized, prevention or therapy at least one medical indication as disclosed herein, comprising performing the method according to the present invention as disclosed herein, and formulating the at least one pharmaceutical drug or combination of pharmaceutical drugs as selected into a suitable pharmaceutical composition.

Pharmaceutical compositions as formulated comprise a pharmaceutically acceptable carrier.

Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for respective compounds, for example topical, and will readily be able to choose suitable pharmaceutically acceptable carriers or excipients, depending, e.g., on the formulation and administration route of the pharmaceutical composition.

The therapeutics can be administered orally, e.g., in the form of pills, tablets, coated tablets, sugar coated tablets, hard and soft gelatin capsules, solutions, syrups, emulsions or suspensions or as aerosol mixtures. Administration, however, can also be carried out rectally, e.g., in the form of suppositories, or parenterally, e.g., in the form of injections or infusions, or percutaneously, e.g., in the form of ointments, creams or tinctures.

In addition to the aforementioned compounds as selected in the present invention, the pharmaceutical composition can contain further customary, usually inert carrier materials or excipients. Thus, the pharmaceutical preparations can also contain additives, such as, for example, fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants. They can also contain the aforementioned salts of two or more compounds as selected in the present invention and also other therapeutically active substances, e.g. as combinations as above.

Similar to the above, preferred is a method according to the present invention, wherein the plurality of medical indications includes at least one of cancer, vascular diseases, lung diseases, brain diseases, metabolic diseases, autoimmune diseases, liver diseases, infectious diseases, such as bacterial or viral infections.

In another aspect of the present invention, the above object is solved by system for determining efficacies for one or more pharmaceutical drugs for a plurality of medical indications, the system comprising
at least on standardized testing system positioned at at least one local testing center, the at least one testing system comprising configured to
receive and/or generate cells from at least one patient-derived tissue sample,
generate 3D microtissues in said system based on the cells as provided,
contact the 3D microtissues as generated with the one or more pharmaceutical drugs to be tested,
collect raw data regarding the effect of the one or more pharmaceutical drugs on the 3D microtissues, and
to transmit the raw data as collected to a centralized server;
a centralized server, connected to said at least on standardized testing system, configured to determine the efficacies for the one or more pharmaceutical drugs as tested for at least one medical indication, and preferably for a plurality of medical indications.

Preferred is a system according to the present invention, further comprising means for selecting the pharmaceutical drug as determined having the highest efficacy for at least one medical indication, wherein more preferably the efficacy is patient-specific. Further preferred is the system according to the present invention, wherein the means are configured to select a combination of pharmaceutical drugs that were determined as having the highest efficacy for at least one medical indication.

Preferred is a system according to the present invention, comprising a plurality (2, 3, 4, or more) of standardized testing systems transmitting raw data as collected to the centralized server, wherein the standardized systems are positioned at the same or different locations.

The standardized testing system comprises means to autonomously generate standardized and thus highly compatible raw data regarding the effect of the one or more pharmaceutical drugs on the 3D microtissues as automatically and autonomously generated.

In order to be perform the methods according to the present invention, the standardized testing system comprises means for handling the media comprising the drugs to be tested, and other consumed materials (well-plates, etc.). Preferably, these materials are supplied as ready-to-use supply packs or so-called preconfigured containers that can be fed into the standardized testing system with minimal handling. In a preferred embodiment, the standardized testing system thus includes suitable means to introduce these packs, like sterile ports, robotic handling arms, a loading system with a lock system, and the like.

Located at the test center, the standardized testing system will then optionally comprise means to put in additional and/or "secondary" data, for example a suitable interface. Again, advantageously this is done with minimal manual handling, for example using a bar code reader reading bar code-encoded test samples. Alternatively, or additionally, the standardized testing system will include means to automatically administer sample material as introduced into the system, in order to exclude mix-ups and other errors.

Preferred is the system according to the present invention, comprising means to transmit the raw data as collected at the (plurality of) separate testing center(s) to the centralized server together with patient-specific secondary data, wherein preferably the patient-specific secondary data comprise one or more of: an electronic health record of the patient, genomic data of the patient, and/or data relating to one or more biomarkers of the patient as analyzed or co-analyzed.

The standardized testing system furthermore comprises means to suitably dissociate the tissue sample and/or cells in said system. Dissociating said tissue sample may comprise means to dissect said tissue sample into smaller pieces comprising cells, means to treat the tissue sample with a solution comprising at least one enzyme capable of dissociating cells in said tissue sample, preferably at least one enzyme selected from a protease, a collagenase, trypsin, elastase, hyaluronidase, papain, chymotrypsin, deoxyribonuclease I, and neutral protease (dispase), producing a supernatant comprising dissociated cells, and means to remove supernatant comprising the dissociated cells and suitably collecting said cells, means to handle the cells between different stages in the process, and preferably means to sonicate the tissue sample with ultrasound.

The standardized testing system furthermore comprises means to generate 3D microtissues in said system based on the cells or tissues as provided. The system as described in, for example, WO 2021/110799, can be readily adjusted to the requirements of the system as described herein, e.g. by providing it with a digital interface to run the individual protocols and transfer the raw or production data to the external data system as herein for further data assessment.

After the 3D microtissues, such as microtumors, have been generated based on the cells or tissues as provided in the system, the 3D microtissues are contacted with the one or more pharmaceutical drugs to be tested. Since the pharmaceutical drugs to be tested are preferably supplied as ready-to-use supply packs or so-called "preconfigured containers" the system comprises means to feed the containers into the standardized testing system with minimal handling. In a preferred embodiment, the standardized testing system thus includes sterile ports, robotic handling arms, a loading system with a lock system, and the like. More preferably, the preconfigured container comprises a multi-well plate, further preferably having pre-defined concentrations of the one or more drugs at predefined locations.

Preferred is the system according to the present invention, comprising means to contact the 3D microtissues with the one or more pharmaceutical drugs to be tested by placing the 3D microtissues at these predefined locations.

In a preferred embodiment, the standardized testing system includes a plurality of the preconfigured containers, each comprising a plurality of receptacles comprising pre-defined concentrations of the one or more drugs at predefined locations. The containers are preferably coded or identifiable with barcodes or the like that are attached. The drugs are as above.

Further preferred is the system according to the present invention, wherein the preconfigured container furthermore comprises a temperature logger configured to log a temperature of the preconfigured container over a, optionally predetermined, period of time, wherein preferably the temperature logger is mounted at a preconfigured container that comprises the preconfigured multi-well plate.

In another embodiment of the system according to the present invention, the preconfigured multi-well plate comprises a cytotoxic agent at one or more predetermined control locations, and preferably the system comprises means for detecting and analyzing whether the cells at the control locations of the preconfigured multi-well plate comprise dead cells at the one or more predetermined control locations as a positive control. Otherwise, the data obtained based on the preconfigured multi-well plate is regarded as invalid.

The system of the present invention importantly comprises means to collect raw data regarding the effect of the one or more pharmaceutical drugs to be tested on the 3D microtissues. Collecting the data regarding the effect may comprise means for determining a physiological parameter selected from a size determination of said 3D microtissue, quantification of internal reporter gene expression in said 3D microtissue, determination of the intracellular ATP content in said 3D microtissue, and determination of pre-selected biomarkers in said 3D microtissue, wherein preferably said size determination of said 3D microtissue comprises at least one parameter selected from diameter, perimeter, volume, and area of optical cross section, and wherein preferably said size determination of said 3D microtissue comprises the use of an imaging device, and optionally further comprises means for analyzing growth-kinetics.

The system of the present invention may be configured as a high-throughput system (HTS), i.e. suitable for performing a multitude of tests or assays in parallel and fully automated.

Further preferred is the system according to the present invention, wherein the standardized testing system is fully integrated, i.e., all components are located in one enclosed housing (provided with the openings as required, e.g. for introducing the materials to be used, see above). The standardized testing system may also comprise two separate units that are functionally connected, one for the cell handling and culture, and the other for the testing of the at least one pharmaceutical drug. Enclosing has the advantage of keeping the system sterile and limiting human interaction.

The system according to the present invention further comprises means for collecting and/or storing of the raw and/or secondary data in at least one local database present in the standardized system present at the local center. The data may be selected from the group consisting of data with respect to the pre-selection and/or grouping of the combination or panel of drugs, physiological parameters, data with respect to the effect(s) of said drugs or combination or panel or group of drugs thereof, data with respect to adverse effects, data with respect to additional clinical parameters, such as patient specific data and/or treatment history, data with respect to the stratification and/or monitoring, and data for automatization of the standardized system, for example for controlling at least one robot thereof. The data may be encrypted.

One or more, and preferably all of the above parameters are then used as raw data and are transmitted from a local database of the standardized system to a centralized server using means for transmitting data of the system. The data may be encrypted for the transfer. The transfer may be directly between the standardized system(s) and the centralized server or via an indirect transfer, e.g., the internet or other network structure. Preferred is the data transfer using a virtual private network (VPN).

In the system of the present invention, the centralized server, based on the data as received comprise means to determine the efficacy for the one or more pharmaceutical drugs as tested for at least one medical indication or for a multitude of medical indication. According to the present invention, the means for determining the efficacy for the one or more pharmaceutical drugs as tested for at least one medical indication or for a multitude of medical indication comprise means for the steps of the introduction of the raw (and secondary) data into the server, a step of curation (organization and integration of data collected from various sources), (further) standardization, and quality control (QC) of the data, before the actual data analysis and interpretation, preferably comprising suitable artificial intelligence (AI) software, and/or machine learning (ML) software (see, for example, Paul D, Sanap G, Shenoy S, Kalyane D, Kalia K, Tekade RK. Artificial intelligence in drug discovery and development. Drug Discov Today. 2021 Jan;26(1):80-93. doi: 10.1016/j.drudis.2020.10.010. Epub 2020 Oct 21. PMID: 33099022; PMCID: PMC7577280; Wang Y, Jeon H. 3D cell cultures toward quantitative high-throughput drug screening. Trends Pharmacol Sci. 2022 Jul;43(7):569-581. doi: 10.1016/j.tips.2022.03.014. Epub 2022 Apr 30. PMID: 35504760).

The centralized server may be linked to other information sources providing additional medical and/or scientific and/or pharmaceutical data ("third party information"), in order to improve and/or complete the determination as required. The third party information may be selected from the group consisting of data with respect to the pre-selection and/or grouping of the combination or panel of drugs, physiological parameters, data with respect to the effect(s) of said drugs or combination or panel or group of drugs thereof, data with respect to adverse effects, data with respect to additional clinical parameters, such as patient specific data and/or treatment history, and data with respect to the stratification and/or medical monitoring.

Before an output is generated from the centralized server, the data processing means may comprise means for performing the additional steps of standardization, quality control (QC) of the output data, and may include automated failure alerts, for example if the quality of the raw data as provided is insufficient in order to determine a statistical meaningful result, as well as means for a respective feedback or feedbacks to operators.

The data processing according to the system according to the present invention may also comprise the use of a data clustering algorithm (see, for example, Pina A, Macedo MP, Henriques R. Clustering Clinical Data in R. Methods Mol Biol. 2020;2051:309-343. doi: 10.1007/978-1-4939-9744-2_14. PMID: 31552636).

Preferably, the output as provided from the centralized server comprises an interactive cloud-based reporting to customers, e.g., as present in the local centers. The output may be direct to the customer, or indirect through suitable means at centralized system(s). Preferred is the data transfer using a virtual private network (VPN). Preferably, the output data is encrypted.

The database and/or data platform may be present on the centralized server itself or a separate server or in the cloud. Preferably, the data in the database is encrypted.

The preferred architecture of the system according to the present invention comprises a centralized server that is centrally connected to each of a multitude of present centralized systems at the local centers, forming a closed or substantially closed network, in order to improve both the safety of the data as generated and the maintenance of the quality of the data as required.

Therefore, preferred is a system for determining efficacies of one or more pharmaceutical drugs to be tested for a plurality of medical indications, the system comprising a plurality of standardized testing systems, each testing system configured to
- obtain cells from a patient-derived sample or obtain cells from a patient-derived tissue sample,
- a plurality of preconfigured containers, each comprising a plurality of receptacles or wells, based on the cells as obtained, generate 3D microtissues in the receptacles of one of the preconfigured containers;
- contact the 3D microtissues with the one or more pharmaceutical drugs to be tested;
- collect raw data regarding the effect of the one or more pharmaceutical drugs on the 3D microtissues,
- transmit the raw data as collected to a centralized server effect data of the one or more drugs on the 3D microtissues, preferably in a standardized format to a server, and
the centralized server, wherein the centralized server is configured to receive the effect data and, based on the received effect data, determine efficacies of the drugs for the medical indication(s).

Another aspect of the present invention then relates to use of the system according to the present invention for generating a database and/or data platform comprising data with respect to the efficacies for the one or more pharmaceutical drugs as tested for a plurality of medical indications, comprising performing the method according to the present invention as above on a centralized server that autonomously determines and generates the data with respect to the efficacies for the one or more pharmaceutical drugs as tested for a plurality of medical indications, and the step of storing said data as generated in a database and/or data platform. Optionally, the database and/or data platform comprising data with respect to the efficacies for the one or more pharmaceutical drugs as tested for a plurality of medical indications further comprise suitable output means.

Another aspect of the present invention then relates to a first computer program, comprising means and being implemented to control the system of the present invention as described herein, i.e., for implementing an improved method for determining efficacies for one or more pharmaceutical drugs for a plurality of medical indications according to the present invention, the first computer program comprising program means configured to obtain raw data regarding the effect of one or more pharmaceutical drugs for a plurality of patients that are afflicted by at least one of the plurality of medical indications, at a plurality of separate testing sites or centers, and using standardized testing systems, wherein said obtaining comprises the steps of providing cells from at least one patient-derived tissue sample, using the standardized testing system, generating 3D microtissues in said system based on the cells as provided; contacting the 3D microtissues as generated with the one or more pharmaceutical drugs to be tested; collecting the raw data regarding the effect of the one or more pharmaceutical drugs on the 3D microtissues; and transmitting the raw data as collected to a centralized server.

Another aspect of the present invention then relates to a second computer program, also comprising means and being implemented to control the system of the present invention as described herein, i.e., for implementing an improved method for determining efficacies for one or more pharmaceutical drugs for a plurality of medical indications according to the present invention, the second computer program comprising program means to, on the centralized server, based on the raw effect data as received at the centralized server, determine the efficacies for the one or more pharmaceutical drugs as tested for at least one medical indication, and preferably for a plurality of medical indications.

In a preferred embodiment, the first and second computer program is combined so that the same software runs on the centralized server and/or the at least one standardized testing systems. Further preferably, the centralized server controls the system of the present invention as described herein. Further preferably, the first and second computer program as well as the combined version allow to run and control the system substantially without human intervention ("automated") as described above.

Another aspect of the present invention then relates to a method of treating a patient that suffers from, or is being diagnosed for, at least one of cancer, vascular diseases, lung diseases, brain diseases, metabolic diseases, autoimmune diseases, liver diseases, infectious diseases, such as bacterial or viral infections in particular a neoplastic disease or tumor, comprising performing the method according to the present invention, and the step of suitably treating said patient with a patient-specific, in particular personalized, drug or drug combination as identified, comprising administering an effective amount of said patient-specific, in particular personalized, drug or drug combination as identified to said patient.

Another aspect of the present invention then relates to a method of stratifying a patient suffering from at least one medical indication, comprising performing the method according to the present invention, and the step of stratifying the patient into at least one treatment group based on the efficacies as determined for the one or more pharmaceutical drugs as tested for at least one medical indication.

Another aspect of the present invention then relates to a method of monitoring the efficacy of the treatment of at least one medical indication in a patient suffering from said at least one medical indication, comprising performing the method according to the present invention on at least two samples obtained from the patient, and the step of monitoring the treatment based on the efficacies as determined for the one or more pharmaceutical drugs as used to treat the at least one medical indication. The patient may suffer from, or is being diagnosed for, at least one of cancer, vascular diseases, lung diseases, brain diseases, metabolic diseases, autoimmune diseases, liver diseases, infectious diseases, such as bacterial or viral infections, in particular a neoplastic disease or tumor.

As mentioned above, compared with the state of the art, the methods and systems according to the present invention have several advantages. Overall, a reliable process is provided that avoids human bias, and thus provides highly significant and comparable data. This ultimately renders both processes of the identification of efficient chemotherapeutic drugs for certain indications as well as the development of more effective and efficient therapies faster, and also cheaper. Since in many proliferative diseases time is of the essence, this will also result in better patient outcome.

Furthermore, while the methods and systems according to the present invention involves are both standardized, autonomous, and "integrated", i.e., largely closed to external interaction that could interfere with the steps of obtaining the raw data, in another preferred embodiment of the method, secondary and/or "foreign" relevant data can be integrated into the analysis as well. Nevertheless, since the core function of the system is largely closed to external interaction, "distortion" or "contamination" of the core functional data is avoided, which maintains the high significance of the data and results as provided.

One preferred example is that after autonomous data generation (e.g. in a 3D Twin Profiler system), the data are being directly wired pseudomized into the cloud without additional manual interaction, and further accessible on by the centralized server as described herein.

The present invention preferably relates to the following items.
Item 1. A method for determining efficacies for one or more pharmaceutical drugs for a plurality of medical indications, the method comprising
   i) obtaining raw data regarding the effect of one or more pharmaceutical drugs for a plurality of patients that are afflicted by at least one of the plurality of medical indications, at a plurality of separate testing sites or centers, and using standardized testing systems,
      wherein said obtaining comprises the steps of:
      - providing cells from at least one patient-derived tissue sample;
      - using the standardized testing system, generating 3D microtissues in said system based on the cells as provided;
      - contacting the 3D microtissues as generated with the one or more pharmaceutical drugs to be tested;
      - collecting the raw data regarding the effect of the one or more pharmaceutical drugs on the 3D microtissues; and
   ii) transmitting the raw data as collected to a centralized server; and
   iii) based on the raw effect data as received at the centralized server, determining the efficacies for the one or more pharmaceutical drugs as tested for at least one medical indication, and preferably for a plurality of medical indications, at the centralized server.
Item 2. The method according to Item 1, further comprising the step of selecting the pharmaceutical drug as determined having the highest efficacy for at least one medical indication, wherein more preferably the efficacy is patient-specific.
Item 3. The method according to Item 1 or 2, further comprising the step of a selecting combination of pharmaceutical drugs as determined having the highest efficacy for at least one medical indication, wherein more preferably the efficacy is patient-specific.
Item 4. The method according to any one of Items 1 to 3, wherein a plurality of standardized testing systems transmits raw data as collected to the centralized server, and wherein the standardized systems are positioned at the same or different locations.
Item 5. The method according to any one of Items 1 to 4, wherein the standardized testing system autonomously generates standardized and highly compatible raw data regarding the effect of the one or more pharmaceutical drugs on the 3D microtissues as automatically generated.
Item 6. The method according to any one of Items 1 to 5, wherein the raw data as collected at the plurality of separate testing centers is transmitted to the centralized server together with patient-specific secondary data, wherein preferably the patient-specific secondary data comprise one or more of: an electronic health record of the patient, genomic data of the patient, and/or data relating to one or more biomarkers of the patient as analyzed or co-analyzed.
Item 7. The method according to any one of Items 1 to 6, wherein the at least one patient-derived tissue sample is selected from a sub-sample derived from a primary tissue sample, a primary tumor sample, and a metastasis sample, wherein preferably said tissue sample has been obtained by a method comprising core biopsy, tumor resection, liquid biopsy and/or needle aspiration, and/or wherein said tissue sample and/or the dissociated cells are frozen and re-thawed prior to the generation of said 3D microtissues.
Item 8. The method according to any one of Items 1 to 7, comprising the step of a dissociation of the tissues and/or cells and the subsequent generation of 3D microtissues in said system.
Item 9. The method according to any one of Items 1 to 8, wherein the pharmaceutical drugs to be tested are supplied as ready-to-use supply packs or preconfigured containers that can be fed into the standardized testing system with minimal handling, preferably comprising a multi-well plate, further preferably having pre-defined concentrations of the one or more drugs at predefined locations.
Item 10. The method according to any one of Items 1 to 9, wherein said contacting may comprises a continuous exposure to the pharmaceutical drugs to be tested and/or combinations thereof, and/or a cycle of an exposure to and subsequent removal to said pharmaceutical drugs to be tested and/or combinations thereof, optionally for at least one, preferably two and more.
Item 11. The method according to any one of Items 1 to 10, wherein the pharmaceutical drugs to be tested or combinations thereof are selected from the group consisting of cytotoxic, cytostatic and/or chemotherapeutic agents, targeted drugs, immunotherapeutic agents and/or combinations thereof.
Item 12. The method according to any one of Items 1 to 11, further comprising the step of analyzing whether the cells at the control locations of the preconfigured multi-well plate comprise dead cells at the one or more predetermined control locations as a positive control.
Item 13. The method according to any one of Items 1 to 12, further comprising the step of collecting and/or storing of the raw and/or secondary data in at least one local database present in the standardized system present at the local center.
Item 14. The method according to any one of Items 1 to 13, wherein the data is patient-specific, i.e., derived from and/or assigned to one patient, and/or one patient sample, such as the 3D microtissue or -tumor, or wherein the data is patient-specific for a group of patients, e.g., different patients suffering from the same type of disease or medical indication, such as a cancer or other proliferative diseases, or a group of patients that have been assigned to the same or substantially the same therapeutic intervention, such as a chemotherapy.
Item 15. The method according to any one of Items 1 to 14, wherein the data processing at the centralized server comprises standardization, quality control (QC) of the output data, and includes automated failure alerts.
Item 16. The method according to any one of Items 1 to 15, further comprising the step of obtaining, at the server, information, such as a feedback, on the therapeutic success of the one or more pharmaceutical drugs.
Item 17. A method for generating a database and/or data platform comprising data with respect to the efficacies for the one or more pharmaceutical drugs as tested for a plurality of medical indications, comprising performing the method according to any one of Items 1 to 16 on a centralized server that autonomously determines and generates the data with respect to the efficacies for the one or more pharmaceutical drugs as tested for a plurality of medical indications, and the step of suitably storing said data as generated in a database and/or data platform.
Item 18. A method for producing a pharmaceutical composition having improved efficacy for, preferably the personalized, prevention or therapy at least one medical indication, comprising performing the method according to any one of Items 1 to 16, and formulating the at least one pharmaceutical drug or combination of pharmaceutical drugs as selected into a suitable pharmaceutical composition.
Item 19. A system for determining efficacies for one or more pharmaceutical drugs for a plurality of medical indications, the system comprising
   at least on standardized testing system positioned at at least one local testing center, the at least one testing system comprising configured to
   receive and/or generate cells from at least one patient-derived tissue sample,
   generate 3D microtissues in said system based on the cells as provided,
   contact the 3D microtissues as generated with the one or more pharmaceutical drugs to be tested,
   collect raw data regarding the effect of the one or more pharmaceutical drugs on the 3D microtissues, and
   to transmit the raw data as collected to a centralized server;
   a centralized server, connected to said at least on standardized testing system, configured to determine the efficacies for the one or more pharmaceutical drugs as tested for at least one medical indication, and preferably for a plurality of medical indications.
Item 20. The system according to Item 19, further comprising means for selecting the pharmaceutical drug as determined having the highest efficacy for at least one medical indication.
Item 21. The system according to Item 19 or 20, wherein the means are configured to select a combination of pharmaceutical drugs that are determined as having the highest efficacy for at least one medical indication.
Item 22. The system according to any one of Items 19 to 21, comprising a plurality of standardized testing systems transmitting raw data as collected to the centralized server, wherein the standardized systems are positioned at the same or different locations.
Item 23. The system according to any one of Items 19 to 22, comprising means to autonomously generate standardized and thus highly compatible raw data regarding the effect of the one or more pharmaceutical drugs on the 3D microtissues as automatically and autonomously generated.
Item 24. The system according to any one of Items 19 to 23, comprising a standardized testing system comprising means to put in additional and/or "secondary" data, for example a suitable interface.
Item 25. The system according to any one of Items 19 to 24, further comprising means to transmit the raw data as collected at the (plurality of) separate testing center(s) to the centralized server together with patient-specific secondary data, wherein preferably the patient-specific secondary data comprise one or more of: an electronic health record of the patient, genomic data of the patient, and/or data relating to one or more biomarkers of the patient as analyzed or co-analyzed.
Item 26. The system according to any one of Items 19 to 25, wherein the standardized testing system includes a plurality of the preconfigured containers, each comprising a plurality of receptacles comprising pre-defined concentrations of the one or more drugs at predefined locations, wherein preferably the containers are coded or identifiable with barcodes or the like that are attached.
Item 27. The system according to Item 26, wherein the preconfigured container furthermore comprises a temperature logger configured to log a temperature of the preconfigured container over a, optionally predetermined, period of time, wherein preferably the temperature logger is mounted at a preconfigured container that comprises the preconfigured multi-well plate.
Item 28. The system according to any one of Items 19 to 27, wherein the system is configured as a high-throughput system (HTS), and preferably fully automated,
Item 29. The system according to any one of Items 19 to 28, wherein the standardized testing system is fully integrated.
Item 30. The system according to any one of Items 19 to 29, further comprising means for collecting and/or storing of the raw and/or secondary data in at least one local database present in the standardized system present at the local center.
Item 31. The system according to any one of Items 19 to 30, wherein the centralized server is linked to other information sources providing additional medical and/or scientific and/or pharmaceutical data.
Item 32. The system according to any one of Items 19 to 31, comprising a centralized server that is centrally connected to each of a multitude of present centralized systems at the local centers, forming a closed or substantially closed network.
Item 33. Use of the system according to any one of Items 19 to 32 for generating a database and/or data platform comprising data with respect to the efficacies for the one or more pharmaceutical drugs as tested for a plurality of medical indications.
Item 34. A computer program, comprising means and being implemented to control the system according to any one of Items 19 to 33, the computer program comprising program means configured to obtain raw data regarding the effect of one or more pharmaceutical drugs for a plurality of patients that are afflicted by at least one of the plurality of medical indications, at a plurality of separate testing sites or centers, and using standardized testing systems, wherein said obtaining comprises the steps of providing cells from at least one patient-derived tissue sample, using the standardized testing system, generating 3D microtissues in said system based on the cells as provided; contacting the 3D microtissues as generated with the one or more pharmaceutical drugs to be tested; collecting the raw data regarding the effect of the one or more pharmaceutical drugs on the 3D microtissues; and transmitting the raw data as collected to a centralized server.
Item 35. A computer program, comprising means and being implemented to control the system according to any one of Items 19 to 3,3 the computer program comprising program means to, on the centralized server, based on the raw effect data as received at the centralized server, determine the efficacies for the one or more pharmaceutical drugs as tested for at least one medical indication, and preferably for a plurality of medical indications.
Item 36. A computer program, comprising a computer program according to Item 34, combined with the computer program according to Item 35.

The present invention will now be described further in the following examples with reference to the accompanying Figures, nevertheless, without being limited thereto. For the purposes of the present invention, all references as cited herein are incorporated by reference in their entireties.

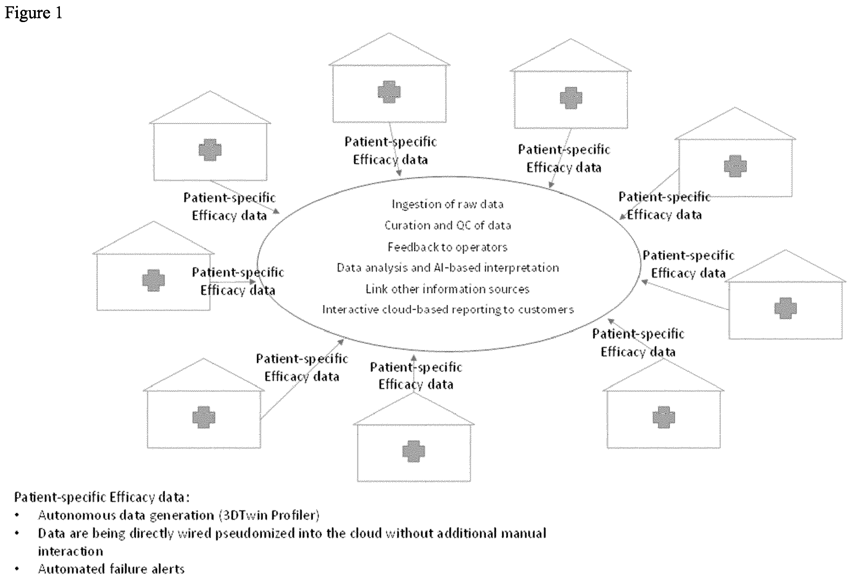

Figure 1 shows a schematic overview of the system
Figure 2 shows eight individual primary image data showing microtumors using different imaging devices (comprising Molecular Device Image express, Molecular Device Pico, Leica DMi6000 and SCREEN Holding Cell3Imager, 3DTwin Profiler). Heterogenous primary data is leading to higher variability in the analysis impeding accuracy of drug response prediction.
Figure 3 shows a comparison of manually B) and automatically A) manufactured microtumors, an error between 6-9% (Fig. 3 A) compared to 5-32% (Fig 3B) for manual production demonstrates the requirement of a standardized and automated process.

### Examples

### Standardization of primary data quality

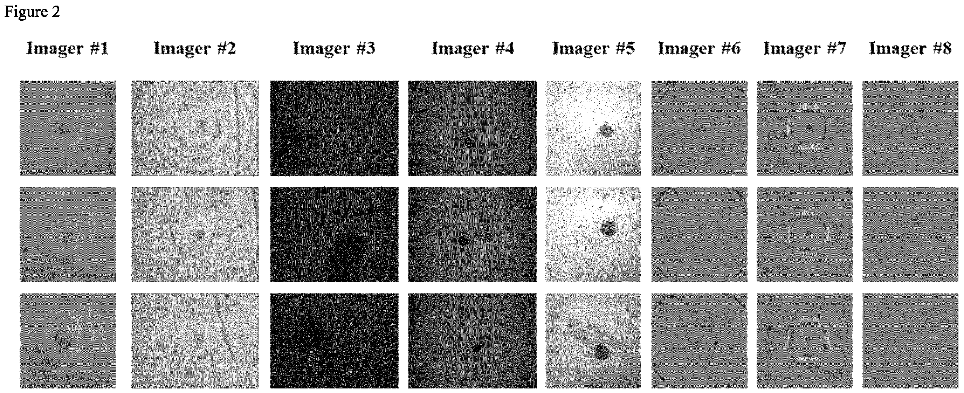

For robust analysis of primary raw data generated at different locations standardized and automated data acquisition is required. Figure 2 shows eight individual primary image data showing microtumors from different imaging devices (comprising Molecular Device Image express, Molecular Device Pico, Leica DMi6000 and SCREEN Holding Cell3Imager, 3DTwin Profiler). Heterogenous primary data is leading to higher variability in the analysis impeding accuracy of drug response prediction.

### Automation - Standardization

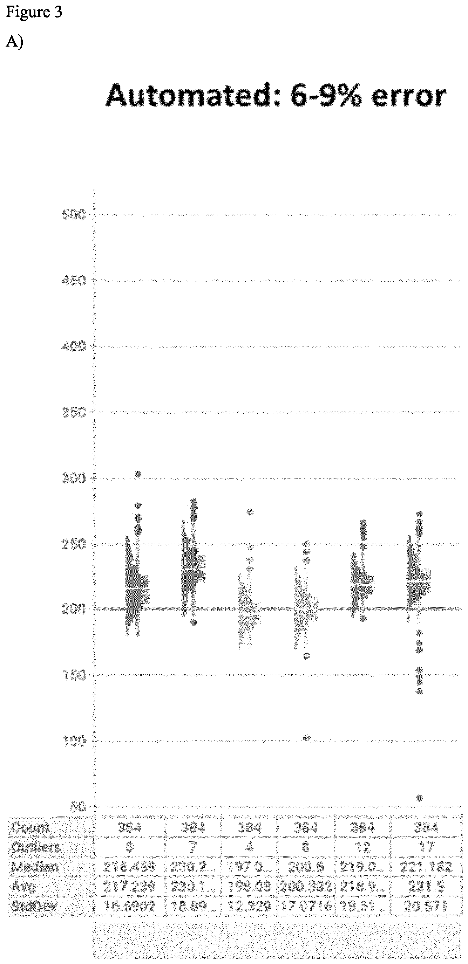

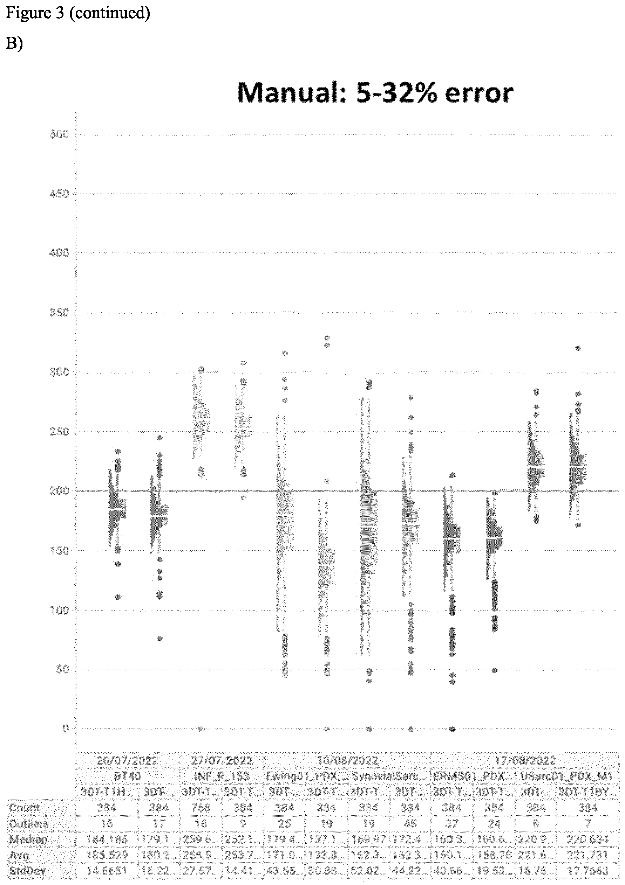

One of the main challenges using next generation sequencing data in the context of big data is standardization (see, for example, Endrullat C, Glökler J, Franke P, Frohme M. Standardization and quality management in next-generation sequencing. Appl Transl Genom. 2016 Jul 1; 10:2-9. doi: 10.1016/j.atg.2016.06.001. PMID: 27668169; PMCID: PMC5025460). Standardization and automation to ensure high data quality and comparable is even more important for complex test systems which utilize living cells where a high number of variables are impacting the results. For cell-based drug response testing this includes, drug format, cell culture medium, culture conditions, drug exposure time, drug concentrations, culture format amongst others. Manual processes lead inherently to variations which are difficult to trace and monitor. Figure 3 shows a comparison of manual and automated manufactured microtumors. Automated processes with an error between 6-9% (Fig. 3 A) compared to 5-32% (Fig 3B) for manual production demonstrates the requirement of a standardized and automated process to create a clinical drug testing network to be able to use the results for big data explorations.

## Claims

1. A method for determining efficacies for one or more pharmaceutical drugs for a plurality of medical indications, the method comprising
i) obtaining raw data regarding the effect of one or more pharmaceutical drugs for a plurality of patients that are afflicted by at least one of the plurality of medical indications, at a plurality of separate testing sites or centers, and using standardized testing systems,
wherein said obtaining comprises the steps of:
- providing cells from at least one patient-derived tissue sample;
- using the standardized testing system, generating 3D microtissues in said system based on the cells as provided;
- contacting the 3D microtissues as generated with the one or more pharmaceutical drugs to be tested;
- collecting the raw data regarding the effect of the one or more pharmaceutical drugs on the 3D microtissues; and
ii) transmitting the raw data as collected to a centralized server; and
iii) based on the raw effect data as received at the centralized server, determining the efficacies for the one or more pharmaceutical drugs as tested for at least one medical indication, and preferably for a plurality of medical indications, at the centralized server, and optionally further comprising the step of selecting the pharmaceutical drug as determined having the highest efficacy for at least one medical indication, wherein more preferably the efficacy is patient-specific.

2. The method according to claim 1, further comprising the step of a selecting combination of pharmaceutical drugs as determined having the highest efficacy for at least one medical indication, wherein more preferably the efficacy is patient-specific.

3. The method according to claim 1 or 2, wherein a plurality of standardized testing systems transmits raw data as collected to the centralized server, and wherein the standardized systems are positioned at the same or different locations, and/or wherein the standardized testing system autonomously generates standardized and highly compatible raw data regarding the effect of the one or more pharmaceutical drugs on the 3D microtissues as automatically generated, and/or wherein the raw data as collected at the plurality of separate testing centers is transmitted to the centralized server together with patient-specific secondary data, wherein preferably the patient-specific secondary data comprise one or more of: an electronic health record of the patient, genomic data of the patient, and/or data relating to one or more biomarkers of the patient as analyzed or co-analyzed.

4. The method according to any one of claims 1 to 3, wherein the pharmaceutical drugs to be tested are supplied as ready-to-use supply packs or preconfigured containers that can be fed into the standardized testing system with minimal handling, preferably comprising a multi-well plate, further preferably having pre-defined concentrations of the one or more drugs at predefined locations.

5. The method according to any one of claims 1 to 4, further comprising the step of analyzing whether the cells at the control locations of the preconfigured multi-well plate comprise dead cells at the one or more predetermined control locations as a positive control.

6. The method according to any one of claims 1 to 5, further comprising the step of collecting and/or storing of the raw and/or secondary data in at least one local database present in the standardized system present at the local center.

7. The method according to any one of claims 1 to 6, wherein the data is patient-specific, i.e., derived from and/or assigned to one patient, and/or one patient sample, such as the 3D microtissue or -tumor, or wherein the data is patient-specific for a group of patients, e.g., different patients suffering from the same type of disease or medical indication, such as a cancer or other proliferative diseases, or a group of patients that have been assigned to the same or substantially the same therapeutic intervention, such as a chemotherapy.

8. The method according to any one of claims 1 to 7, wherein the data processing at the centralized server comprises standardization, quality control (QC) of the output data, and includes automated failure alerts.

9. The method according to any one of claims 1 to 8, further comprising the step of obtaining, at the server, information, such as a feedback, on the therapeutic success of the one or more pharmaceutical drugs.

10. A method for generating a database and/or data platform comprising data with respect to the efficacies for the one or more pharmaceutical drugs as tested for a plurality of medical indications, comprising performing the method according to any one of claims 1 to 9 on a centralized server that autonomously determines and generates the data with respect to the efficacies for the one or more pharmaceutical drugs as tested for a plurality of medical indications, and the step of suitably storing said data as generated in a database and/or data platform.

11. A method for producing a pharmaceutical composition having improved efficacy for, preferably the personalized, prevention or therapy at least one medical indication, comprising performing the method according to any one of claims 1 to 9, and formulating the at least one pharmaceutical drug or combination of pharmaceutical drugs as selected into a suitable pharmaceutical composition.

12. A system for determining efficacies for one or more pharmaceutical drugs for a plurality of medical indications, the system comprising
at least on standardized testing system positioned at at least one local testing center, the at least one testing system comprising configured to
receive and/or generate cells from at least one patient-derived tissue sample,
generate 3D microtissues in said system based on the cells as provided,
contact the 3D microtissues as generated with the one or more pharmaceutical drugs to be tested,
collect raw data regarding the effect of the one or more pharmaceutical drugs on the 3D microtissues, and
to transmit the raw data as collected to a centralized server;
a centralized server, connected to said at least on standardized testing system, configured to determine the efficacies for the one or more pharmaceutical drugs as tested for at least one medical indication, and preferably for a plurality of medical indications, optionally further comprising means for selecting the pharmaceutical drug as determined having the highest efficacy for at least one medical indication, wherein preferably the means are configured to select a combination of pharmaceutical drugs that are determined as having the highest efficacy for at least one medical indication and/or comprising a plurality of standardized testing systems transmitting raw data as collected to the centralized server, wherein the standardized systems are positioned at the same or different locations, and/or comprising means to autonomously generate standardized and thus highly compatible raw data regarding the effect of the one or more pharmaceutical drugs on the 3D microtissues as automatically and autonomously generated.

13. The system according to claim 12, further comprising means to transmit the raw data as collected at the (plurality of) separate testing center(s) to the centralized server together with patient-specific secondary data, wherein preferably the patient-specific secondary data comprise one or more of: an electronic health record of the patient, genomic data of the patient, and/or data relating to one or more biomarkers of the patient as analyzed or co-analyzed, wherein preferably the preconfigured container furthermore comprises a temperature logger configured to log a temperature of the preconfigured container over a, optionally predetermined, period of time, wherein preferably the temperature logger is mounted at a preconfigured container that comprises the preconfigured multi-well plate, optionally further comprising means for collecting and/or storing of the raw and/or secondary data in at least one local database present in the standardized system present at the local center, and/or wherein the centralized server is linked to other information sources providing additional medical and/or scientific and/or pharmaceutical data and/or comprising a centralized server that is centrally connected to each of a multitude of present centralized systems at the local centers, forming a closed or substantially closed network.

14. Use of the system according to any one of claims 12 or 13 for generating a database and/or data platform comprising data with respect to the efficacies for the one or more pharmaceutical drugs as tested for a plurality of medical indications.

15. A computer program i), comprising means and being implemented to control the system according to claim 12 or 13, the computer program comprising program means configured to obtain raw data regarding the effect of one or more pharmaceutical drugs for a plurality of patients that are afflicted by at least one of the plurality of medical indications, at a plurality of separate testing sites or centers, and using standardized testing systems, wherein said obtaining comprises the steps of providing cells from at least one patient-derived tissue sample, using the standardized testing system, generating 3D microtissues in said system based on the cells as provided; contacting the 3D microtissues as generated with the one or more pharmaceutical drugs to be tested; collecting the raw data regarding the effect of the one or more pharmaceutical drugs on the 3D microtissues; and transmitting the raw data as collected to a centralized server, or
a computer program ii), comprising means and being implemented to control the system according to claim 12 or 13, the computer program comprising program means to, on the centralized server, based on the raw effect data as received at the centralized server, determine the efficacies for the one or more pharmaceutical drugs as tested for at least one medical indication, and preferably for a plurality of medical indications, or
a computer program iii), comprising a computer program according to i), combined with the computer program according to ii).
